# EUROPEAN PATENT SPECIFICATION

(11) **EP 1 996 308 B1**
(45) Date of publication and mention of the grant of the patent: **21.07.2010**
(21) Application number: 06706113.5
(22) Date of filing: 13.03.2006
(51) Int. Cl.: B01D 33/64, C12P 7/10, B30B 9/10, B30B 9/18

(54) **A METHOD AND A SEPARATING PRESS FOR USE IN THE PRODUCTION OF BIOETHANOL**
VERFAHREN UND TRENNPRESSE ZUR VERWENDUNG BEI DER HERSTELLUNG VON BIOETHANOL
PROCEDE ET PRESSE DE SEPARATION A UTILISER DANS LA FABRICATION DE BIOETHANOL

(43) Date of publication of application: 03.12.2008
(73) Proprietor: J.S. Maskinfabrik A/S, 9800 Hjorring (DK)
(72) Inventor: SøRENSEN, Freddy, DK-9800 Hjørring (DK)
(74) Representative: Larsen, Hans Ole
(86) International application number: PCT/DK2006/000142
(87) International publication number: WO 2007/104305

(56) References cited:
- WO-A-03/086740
- WO-A2-02/38787
- JP-A- 2002 160 092
- JP-A- 2004 181 511
- JP-A- 2006 055 819

## Description

### Summary of the invention

The invention relates to a method of producing bioethanol on the basis of ground plant material, such as corn, said material being suspended in water and heated to above the gelatinization temperature, following which a heat stable α-amylase is added, said mixture being boiled to form a mixture having a high dry matter content, following which glycoamylase and yeast are added to said mixture to form ethanol by distillation, as well as a separating press for use in the performance of the method.

### The prior art

In the previously known methods of producing ethanol on the basis of plant material, such as corn and sugar cane, etc., this preferably takes place by a dry production, i.e, dry grinding to a particle size of about 1 mm.

This granulate is then suspended in water, which is heated to max. 90 °C to get above the gelatinization temperature of the starch content.

Then, a heat stable α-amylase is added, which hydrolyzes the starch to reduce the viscosity thereby.

The mixture is then boiled at a temperature of max. 120 °C, whereby the starch in the liquid will be converted into oligosaccharides.

It is intended to ensure a high dry matter content in the mash to achieve the maximum capacity thereby (very high gravity technology).

The next process step is to decompose saccharides into glycose, which is converted into ethanol by addition of yeast.

Finally, this ethanol is distilled off, following which the insoluble parts of the sediment are removed.

This takes place in either a decanter or a centrifuge in order for the liquid in the sediment to be utilized, preferably by supply to the suspension process step,

Known separators, however, are vitiated by the drawback that they are not very effective in a continuous process, but are solely intended for a single process step, and to this should be added that they are every expensive in operation because of a great consumption of energy. Moreover, they require much maintenance and cleaning, and, finally, the dry matter content, or in other words the degree of separated liquid, is not sufficient to cover the costs of operation. Therefore, the utilization rate in the production of ethanol is not optimum.

An example of a separator for pressing liquid out of fish meat is shown in WO03/086740 by Freddy Sorensen. This separator comprises a shaft with a screw worm and is surrounded by a shell with holes. The fish meat is introduced via inlet openings in the shell and is pressed by the rotating movement as well as by a valve which is placed before the outlet. Similar worm presses are disclosed in JP 2002-160092 A1 by Mitsubishi and JP 2004-181511 A1 by Kubota.

An example of a process of producing bioethanol is shown in WO02/38787 by Christopher Veit. In this method the ethanol is produced in a fermentation process where a ground plant material is suspended in water and heated where after alfa-amylase and glycoamylase is added followed by a distillation process. In order to separate liquid from the residue from the distillation process, the residue is placed in a drum and the liquid is separated by centrifugal forces.

### The object of the invention

It is the object of the invention to improve the production process, and this is achieved according to the invention in that the insoluble parts of the distillation residue, the sediment, are fed to a continuous separating worm press through a channel (10) in the shaft (4) on which a screw worm (5) is mounted and into a press chamber (11) in the interior of a cylindrical screen shell (2a-2c) which make up the press chamber (11), and where the worm press by its rotation against an outlet valve with a cylindrical body (17), also mounted on the shaft (4), and a seat (18) on the valve body (17) and a cooperating seat (15) on the rear edge of the cylindrical screen (2a-2c), which are bevelled each in two or more angles which are mutually parallel, and where the cylindrical body (17) utilizes a variable and pulsating compression pressure on the sediment and thereby separate liquid from the sediment.

Hereby, the utilization rate is optimum, since the dry matter content is very high, and as much liquid as possible is utilized in the production process to form ethanol.

It has surprisingly been found that the pulsation established during the pressing, gives an unprecedentedly high expulsion of liquid, which is related to the effect which is achieved when a hard compression is followed by a less hard compression, whereby the liquid is pulled out of the mass, is released and can be separated and discharged.

When, as stated in claim 2, a continuous separating press being characterized in that the press further comprises a channel (10) in the shaft (4) providing a passage from an inlet opening to the interior of the screen shell (2a-2c), which channel is parallel to the axis of the shaft (4) and has a bend; where the outlet valve (15, 18) has a cylindrical valve body (17) which is mounted on the shaft and extends in 360 degrees around the shaft, and is controlled to exert a pulsating counter-pressure on the sediment in the press chamber (11), and where the valve seat (18) on the valve body (17) and the cooperating seat (15) on the rear edge of the cylindrical screen (2a-2c) are beveled each in two or more angles which are mutually parallel it is ensured that a continuous process may be carried out.

When the valve body is configured such that it performs a cycle of movement which alternately opens and restricts the discharge opening, it is possible to control the process by scanning the absorption of energy of the drive motors and thereby ensure specific valve positions to achieve the desired pressure conditions.

The valve seats are configured such that the flow courses are uniform in the various valve positions, an even flow through the press is achieved.

Finally, as stated in claim 3, it is expedient to provide the valve body with a sealing ring, since a completely closed valve position may be achieved hereby.

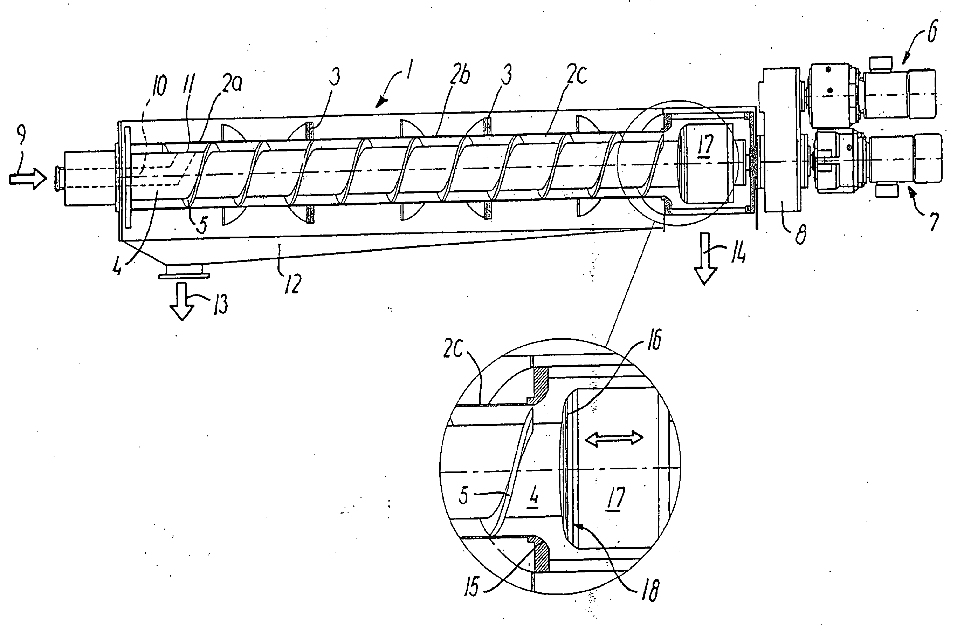

### The drawing

An example of a separating press according to the invention will be described more fully below with reference to the drawing, which shows a partially sectional view of a press and an enlarged view of the valve part.

### Description of the exemplary embodiment

A separating press, which is suitable for performing the method of producing ethanol by expulsion of liquid from the sediment after the actual distillation for the separation of the ethanol has been performed, will be described more fully below.

The press itself is formed by one or more cylindrical screen shells 2a-2c, which are joined adjacently to form a cylinder by means of flanges 3.

The screen shells may have different screen openings, e.g. openings of 0.2 mm in the first shell 2a and openings of 30 µm in the last shell 2c.

Supply of the sediment 9 takes place to the interior of the screen shell, in which a worm 5 on a shaft 4 rotates. The sediment 9 is hereby fed into the press chamber 11, which extends between the screen shells 2a-2c and the shaft 4 and the worm turn 5 to advance the sediment toward the discharge opening 14 at the opposite end of the press.

A valve is mounted at the discharge end of the press chamber 11, said valve being shown more clearly in the enlarged view.

The valve body 17 itself is provided with a seat 18 which is bevelled in one or more angles to form an obliquely extending discharge.

The valve body 17 cooperates with a valve seat 15 on the rear edge of the screen shell 2c, said seat 15 being likewise bevelled corresponding to the cooperating seat 18.

A sealing ring 16 is shown on the valve seat 18, which sealing ring will block the discharge from the press chamber 11 at a closed valve position.

The valve body 17 may be adjusted by means of working cylinders (not shown) so that it may be displaced in the direction of the double arrow, i.e. for engagement and opening.
The screen shells 2a-2c are rotated with a few revolutions per minute in one direction by means of a motor 6 via a gear 8, and the worm 4, 5 rotates in the opposite direction by means of another motor 7 and gear 8, likewise with a few revolutions per minute.

Finally, a collection chute 12 is provided for the collection of liquid, and a discharge is provided for the discharge of liquid 13 during the pressing.

The method of expelling liquid from the sediment will now be explained.

The sediment with a shown liquid content, after the ethanol has been distilled off, is fed via an inner channel 10 to the press chamber 11, as indicated by an arrow 9.

At the beginning, the valve 17 is closed against discharge so that the turns on the worm 5 will advance the sediment to the right in the drawing when the worm is started.

During this feeding, the sediment is compressed, as air and liquid are expelled through the screen openings.

When a suitable period of time has elapsed, a pressure has been generated, and the sediment will engage the valve 17, following which the pulsation of the valve body is initiated, viz. by a movement in a forward and rearward direction.

This pulsation contributes to expelling the liquid, which is collected at the bottom and is discharged, following which the liquid may be returned to the soaking step of the process.

It is this pulsation and switching between a higher and a lower pressure which contributes to an unprecedentedly high efficiency, which takes place during the continuous feeding of sediment and expulsion of liquid. When the pressure drops, the liquid in the sediment tends to flow from the compressed sediment to be pressed out of the sediment as liquid in the subsequent compression.

The valve movement may be controlled by the absorption of energy of the motors 6,7, as the absorption of energy of the motors and the degree of compression will be related.

## Claims

1. A method of producing bioethanol on the basis of ground plant material, such as corn, said material being suspended in water and heated to above the gelatinization temperature, following which a heat stable α-amylase is added, said mixture being boiled to form a mixture having a high dry matter content, following which glycoamylase and yeast are added to said mixture to form ethanol by distillation, **characterized in that** the insoluble parts of the distillation residue, the sediment, are fed to a continuous separating worm press through a channel (10) in the shaft (4) on which a screw worm (5) is mounted and into a press chamber (11) in the interior of a cylindrical screen shell (2a-2c) which make up the press chamber (11), and where the worm press the sediment by its rotation against a discharge valve with a cylindrical body (17), also mounted on the shaft (4), and a seat (18) on the valve body (17) and a cooperating seat (15) on the rear edge of the cylindrical screen (2a-2c), which are bevelled each in two or more angles which are mutually parallel, and where the cylindrical body (17) utilizes a variable and pulsating compression pressure on the sediment and thereby separate liquid from the sediment.

2. A continuous separating press for performing the method according to claim 1, where the press comprises:
- a shaft (4) on which a screw worm (5) is mounted and which is intended to be rotated in one direction by a motor (7);
- a cylindrical rotatable screen shell (2a-2c) which is concentric with the shaft (4), and is assembled by several cylindrical shells (2a-2c);
- where the space between the shaft (4) and the screen shell (2a-2c) make up the press chamber (11) into which the mash is intended to be fed;
- a valve body (17) comprising a valve seat (18), which cooperates with a cooperating valve seat (15) on the rear edge of the cylindrical screen shell (2a-2c) and hereby constituting an outlet valve (15, 18), which is intended to exert a variable counter-pressure on sediment in a press chamber (11) during the separation,
**characterized in that** the press further comprises:
- a channel (10) in the shaft (4) providing a passage from an inlet opening to the interior of the screen shell (2a-2c), which channel is parallel to the axis of the shaft (4) and has a bend;
where the outlet valve (15, 18) has a cylindrical valve body (17) which is
- mounted on the shaft and extends in 360 degrees around the shaft, and
- is controlled to exert a pulsating counter-pressure on the sediment in the press chamber (11), and
- where the valve seat (18) on the valve body (17) and the cooperating seat (15) on the rear edge of the cylindrical screen (2a-2c) are beveled each in two or more angles which are mutually parallel.

3. A continuous separating press according to claim 2, where the valve seat (18) is additionally provided with a sealing ring (16) to engage the seat (15) at the closing of the outlet opening (19).

4. A continuous separating press according to claim 2 or 3, where each of the cylindrical shells (2a-2c) have openings, which the separated liquid can flow out of and where the first shell piece (2a) has openings with a size of up to 0.2 mm and the last shell piece (2c) has openings of up to 0.03 mm such that the size of the holes decrease as a function of the distance from the inlet.

5. A continuous separating press according to any of claims 2-4, where flanges (3) are placed on the cylindrical shells (2a-2c) so that the shells are held together.

## Patentansprüche

1. Verfahren zum Herstellen von Bioethanol auf Basis von gemahlenem Pflanzenmaterial, wie beispielsweise Mais, wobei das Material in Wasser suspendiert und über die Gelatinierungstemperatur erhitzt wird, und anschließend eine wärmestabile α-Amylase zugesetzt wird, das Gemisch zum Kochen gebracht wird, um ein Gemisch mit einem höheren Trockensubstanzgehalt herzustellen, und dann dem Gemisch Glycoamylase sowie Hefe zugesetzt werden, um durch Destillation Ethanol herzustellen, **dadurch gekennzeichnet, dass** die unlöslichen Teile des Destillationsrückstandes, das Sediment, in eine kontinuierliche Trenn-Schneckenpresse über einen Kanal (10) in der Welle (4), an der eine Schnecke (5) angebracht ist, und in eine Presskammer (11) im Inneren einer zylindrischen Siebhülse (2a-2c) eingeleitet werden, die die Presskammer (11) bildet, und wobei die Schnecke das Sediment durch ihre Drehung an ein Ausgabeventil mit einem zylindrischen Körper (17) presst, das ebenfalls an der Welle (4) angebracht ist, und einen Sitz (18) an dem Ventilkörper (17) und ein damit zusammenwirkender Sitz (15) an der hinteren Kante des zylindrischen Siebes (2a-2c), die jeweils in zwei oder mehr Winkeln abgeschrägt sind, die parallel zueinander sind, und wobei der zylindrische Körper (7) einen variablen und pulsierenden Kompressionsdruck auf das Sediment nutzt, um so Flüssigkeit von dem Sediment zu trennen.

2. Kontinuierliche Trennpresse zum Durchführen des Verfahrens nach Anspruch 1, wobei die Presse umfasst:
eine Welle (4), an der eine Schnecke (5) angebracht ist und die von einem Motor (7) in einer Richtung gedreht werden soll;
eine zylindrische drehbare Siebhülse (2a-2c), die konzentrisch zu der Welle (4) ist und die durch mehrere zylindrische Hülsen (2a-2c) gebildet wird;
wobei der Raum zwischen der Welle (4) und der Siebhülse (2a-2c) die Presskammer (1) bildet, in die die Maische eingeleitet werden soll;
einen Ventilkörper (17), der einen Ventilsitz (18) umfasst, der mit einem zusammenwirkenden Ventilsitz (15) an der Hinterkante der zylindrischen Siebhülse (2a-2c) zusammenwirkt und damit ein Auslassventil (15, 18) bildet, das während der Trennung einen variablen Gegendruck auf Sediment in einer Presskammer (11) ausüben soll,
**dadurch gekennzeichnet, dass** die Presse des Weiteren umfasst:
einen Kanal (10) in der Welle (4), der einen Durchlass von einer Einlassöffnung in den Innenraum der Siebhülse (2a-2c) bildet, wobei der Kanal parallel zu der Achse der Welle (4) ist und eine Biegung aufweist;
wobei das Auslassventil (15, 18) einen zylindrischen Ventilkörper (17) hat, der an der Welle angebracht ist und sich um 360 Grad um die Welle herum erstreckt, und
so gesteuert wird, dass er einen pulsierenden Gegendruck auf das Sediment in der Presskammer (11) ausübt, und
wobei der Ventilsitz (18) an dem Ventilkörper (17) und der zusammenwirkende Sitz (15) an der Hinterkante des zylindrischen Siebs (2a-2c) jeweils in zwei oder mehr Winkeln abgeschrägt sind, die parallel zueinander sind.

3. Kontinuierliche Trennpresse nach Anspruch 2, wobei der Ventilsitz (18) des Weiteren mit einem Dichtungsring (16) versehen ist, der beim Schließen der Auslassöffnung (19)mit dem Sitz (15) in Eingriff kommt.

4. Kontinuierliche Trennpresse nach Anspruch 2 oder 3, wobei jede der zylindrischen Hülsen (2a-2c) Öffnungen hat, über die die abgeschiedene Flüssigkeit ausströmen kann, und das erste Hülsenteil (2a) Öffnungen mit einer Größe von bis zu 0,2 mm hat und das letzte Hülsenteil (2c) Öffnungen mit einer Größe bis zu 0,03 mm hat, so dass die Größe der Löcher als Funktion des Abstandes zu dem Einlass abnimmt.

5. Kontinuierliche Trennpresse nach einem der Ansprüche 2-4, wobei Flansche (3) an den zylindrischen Hülsen (2a-2c) angeordnet sind, so dass die Hülsen zusammengehalten werden.

## Revendications

1. Procédé pour produire du bioéthanol sur la base d'une matière de plante de sol, telle que du maïs, ladite matière étant suspendue dans l'eau et chauffée au-dessus de la température de gélatinisation, suite à quoi une alpha-amylase stable chaude est ajoutée, ledit mélange étant porté à ébullition pour former un mélange ayant une teneur en matière sèche élevée, suite à quoi on ajoute de la glycoamylase et de la levure audit mélange afin de former de l'éthanol par distillation, **caractérisé en ce que** les parties insolubles du résidu de distillation, le sédiment, sont alimentées à une presse de séparation à vis sans fin continue, par le biais d'un canal (10), dans l'arbre (4) sur lequel une vis sans fin (5) est montée et dans une chambre de presse (11) dans l'intérieur d'une coque formant tamis cylindrique (2a-2c) qui compose la chambre de presse (11), et où la vis sans fin comprime le sédiment par sa rotation contre une soupape de décharge avec un corps cylindrique (17), également monté sur l'arbre (4), et un siège (18) sur le corps de soupape (17) et un siège coopérant (15) sur le bord arrière du tamis cylindrique (2a-2c), qui sont chacun biseautés sur deux ou plusieurs angles qui sont mutuellement parallèles, et où le corps cylindrique (17) utilise une pression de compression variable et à pulsation sur le sédiment, et sépare ainsi le liquide du sédiment.

2. Presse de séparation continue pour réaliser le procédé selon la revendication 1, dans lequel la presse comprend :
un arbre (4) sur lequel une vis sans fin (5) est montée et qui est prévue pour être entraînée en rotation dans une direction par un moteur (7) ;
une coque rotative formant tamis cylindrique (2a-2c) qui est concentrique par rapport à l'arbre (4), et est assemblée par plusieurs coques cylindriques (2a-2c) ;
dans laquelle l'espace entre l'arbre (4) et la coque formant tamis (2a-2c) compose la chambre de presse (11) dans laquelle on prévoit d'alimenter la trempe ;
un corps de soupape (17) comprenant un siège de soupape (18) qui coopère avec un siège de soupape (15) coopérant sur le bord arrière de la coque formant tamis cylindrique (2a-2c) et constituant ainsi une soupape de sortie (15, 18), qui est prévue pour exercer une contre-pression variable sur le sédiment dans la chambre de presse (11) pendant la séparation,
**caractérisée en ce que** la presse comprend en outre ;
un canal (10) dans l'arbre (4) fournissant un passage à partir de l'ouverture d'entrée jusqu'à l'intérieur de la coque formant tamis (2a-2c), lequel canal est parallèle à l'axe de l'arbre (4) et a un coude ;
dans laquelle la soupape de sortie (15, 18) a un bord de soupape cylindrique (17) qui est :
monté sur l'arbre et s'étend à 360 degrés autour de l'arbre, et
est contrôlé pour exercer une contre-pression de pulsation sur le sédiment dans la chambre de presse (11), et
dans laquelle le siège de soupape (18) sur le corps de soupape (17) et le siège coopérant (15) sur le bord arrière du tamis cylindrique (2a-2c) sont biseautés chacun sur deux ou plusieurs angles qui sont mutuellement parallèles.

3. Presse de séparation continue selon la revendication 2, dans laquelle le siège de soupape (18) est fourni de plus avec une bague d'étanchéité (16) pour mettre en prise le siège (15) lors de la fermeture de l'ouverture de sortie (19).

4. Presse de séparation continue selon la revendication 2 ou 3, dans laquelle chacune des coques cylindriques (2a-2c) ont des ouvertures par lesquelles le liquide séparé peut s'écouler et où la première pièce de coque (2a) a des ouvertures avec une taille allant jusqu'à 0,2 mm et la dernière pièce de coque (2c) a des ouvertures allant jusqu'à 0,03 mm de sorte que la taille des trous diminue en fonction de la distance par rapport à l'entrée.

5. Presse de séparation continue selon l'une quelconque des revendications 2 à 4, dans laquelle on place des rebords (3) sur les coques cylindriques (2a-2c) de sorte que les coques sont maintenues ensemble.
